# Europäisches Patentamt
# European Patent Office
# Office européen des brevets

(11) Publication number: **0 012 122**
**B1**

(12) **EUROPEAN PATENT SPECIFICATION**

(45) Date of publication of patent specification: **04.08.82**

(51) Int. Cl.³: **A 61 N 1/04**

(21) Application number: **79850097.1**

(22) Date of filing: **05.11.79**

(54) **Electrical stimulator.**

(30) Priority: **27.11.78 SE 7812159**

(43) Date of publication of application:
**11.06.80 Bulletin 80/12**

(45) Publication of the grant of the patent:
**04.08.82 Bulletin 82/31**

(84) Designated Contracting States:
**AT BE CH DE FR GB IT LU NL**

(56) References cited:
**DE - A - 2 502 620**
**FR - A - 2 215 978**
**US - A - 749 100**
**US - A - 1 827 306**
**US - A - 3 650 276**

(73) Proprietor: **Crafon Medical AB**
**Box 10101**
**S-220 10 Lund (SE)**

(72) Inventor: **Carlsson, Per-Olov Arne Vilhelm**
**Drottninggatan 4**
**S-280 10 Sösdala (SE)**
Inventor: **Hakansson, Bo Hakan**
**Astrakanvägen 6**
**S-223 56 Lund (SE)**

(74) Representative: **Boberg, Gunnar**
**HOLGER CRAFOORD AB Patent Department Box**
**10101**
**S-220 10 Lund (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Electrical stimulator

Technical field

The present invention relates to an electrical stimulator comprising an element intended for vaginal placing carrying two or more electrodes which are adapted so as to control through electrical pulses from a pulse generator the urethral and/or urocystic functions, said element having a shape that is not rotational-symmetrical.

Background art

Disturbances in the urinary and rectal functions are a great problem for the individual person as well as for the nursing attendant. Urinary incontinence is very customary and is from a nursing point of view very time-consuming in view of the changing of clothing, bedding, etc.

It has been tried to solve these problems in a number of different ways, for example, with the help of technical means for the collecting of the urine which works tolerably satisfactory with men, but which is more difficult to realize with women.

Furthermore, it has been tried to stimulate by electrical means the pelvis floor through the implantation of electrodes in the pelvis floor musculature connected to a pulse generator placed subcutaneously, which can be supplied, e.g. electromagnetically with pulses from a transmitter placed outside the body. This method requires a surgical intervention and is relatively complicated and costly. Examples of such implantable devices are disclosed for example in US patents 3 543 761, 3 667 477 and 3 870 051.

Another method is based on achieving contraction of the urethra by means of electrical stimulation with the help of electrodes arranged on an element carried intravaginally or anally. In known arrangements of this kind generally elements made of a rigid plastic material of circular cross-section are used, the electrodes frequently being constituted or peripheral metal rings. Examples of such devices are described in British patent 1 286 075 and US patent 3 943 938. Apart from the discomfort experienced in the wearing of such a hard element inside the body, there is a risk of it sliding out. Moreover, a stimulation of the whole pelvis region is obtained through the peripheral electrodes, which is not always appropriate.

In the US patent 4 106 511 an improved design of a stimulator of the type mentioned in the introduction is described. This electrical stimulator, which is intended for vaginal or anal placing, comprises an element consisting of a flexible material which is expandable so as to achieve a secure attachment of the element in the body. This element is provided with a substantially plane end wall at the inner end of the element in its applied position. This end wall facilitates the placing and the retention in the desired position. In spite of a not quite rotational-symmetrical shape of the element there is a great risk that it may turn and as a result two groups of electrodes pointing in the same direction may obtain an undesirable direction of stimulation. This tendency of spinning away from the desired position is at first hand due to the fact that the electrodes are placed opposite one or more protuberances intended to facilitate the maintainance of the element in a vaginal or an anal position. A normal vagina has however a cross-section form with a longer dimension at right angles to the longest dimensions of the cross-sections of the known element, providing automatically the above mentioned tendency of spinning.

Disclosure of invention

It is the object of the present invention to provide a stimulator of the type mentioned in the introduction and more parcicularly an improved design compared with the last mentioned design, which in spite of its disadvantages is probably the best of its kind produced up to now.

The improvement consists primarily in that the said element has been given a shape that is not rotational-symmetrical in a different way in that the cross-sections of said element at right angles to an axis which runs parallel with the direction of introduction have an elongated form, the electrodes being arranged on one major surface only of the element on one side of the longitudinal axis of said cross-sections, the shape of the element enabling it to be placed in the vagina in a rotationally-stable position with the electrodes directed towards the bladder. In this way a risk of the element rotating during application, and directing the stimulations towards parts which are not intended to be stimulated, is substantially eliminated. In this connection it should be noted that it has been asserted, for example, that electrical pulses intended to control the urethral or urocystic function may instead control the rectal function if they are pointed in the wrong direction, even if vaginally placed.

The said element is preferably shaped so that it is of substantially oval cross-section at right angles to an axis which runs parallel with the intended direction of introduction, the area of these cross-sections preferably increasing in the direction of introduction. In this manner additional security of retention in the desired position is achieved.

In the design with the abovementioned oval cross-section the component has two less curved surfaces. The electrodes are appropriately arranged on one of these surfaces, which is located in the vagina in a specific manner. In

**0012122**

this manner it is ensured that the electrodes are effective in the intended direction.

Likewise, in the known design described previously the element that can be introduced into the vagina is appropriately designed so that it is expandable by inflation. In this way the introduction is facilitated and the retention is further improved.

The said element is appropriately constituted of a bladder made of an expandable material such as rubber, or plastics, and of a preferably rotation-symmetrical end piece which is provided with a smaller opening for introduction of leads connected to the electrodes, and for the connection of a tube for the inflation and evacuation respectively of the element. This facilitates the manufacture of the element at the same time as allowing it to be made impermeable to gas and liquid.

As an example of a material for the bladder a thermoplastic rubber marketed under the name KRATON (registered trade mark) by Dry Color AB may be mentioned. Alternatively, a silicone rubber or a natural rubber may be used.

The said electric leads are suitably attached to plates which in turn are attached to the shanks of rivetlike electrodes with point effect, whose heads are arranged on the outside of the said element, the shanks penetrating the wall of the element. This allows effective sealing to be achieved, in that the said wall is held clamped between the plates and the heads. This seal can be improved further by giving the said heads and/or the said plates a dished shape facing towards the well of the element.

The shanks of the rivetlike electrodes appropriately have a hollow end to facilitate assembling of the same. The control of the same during assembling is very important in order to be able to ensure sealing. This sealing can be improved further in that the end piece is provided with a circular-cylindrical flange which is attached inside the bladder and a substantially conical flange which is adapted so that it surrounds the mouth of the bladder in a sealing manner.

It should be noted that the seal is of very great importance. If the element used is not impermeable to liquid, some liquid might penetrate into it and e.g. form bacterial centres. On the other hand, if it is not impermeable to gas, the enclosed gas would rapidly be squeezed out of the element, thus reducing the retention, so that there would be a risk of the direction of stimulation being altered or of the patient dropping the element altogether.

Brief description of drawings

The invention is described in more detail in the following with reference to the enclosed drawings, which by way of example describe a preferred embodiment of the same.

Fig. 1 shows the stimulator in its entirety.

Fig. 2 shows a longitudinal section through a bladder forming part of the stimulator.

Fig. 3 shows the same bladder seen from the right in Fig. 2.

Fig. 4 shows the same bladder seen from below according to Fig. 2.

Fig. 5 shows an axial section through an end piece forming part of the stimulator.

Fig. 6 shows the end piece seen from above according to Fig. 5.

Fig. 7 finally shows a rivetlike electrode forming part of the stimulator and its attachment together with its electrical connection.

Preferred embodiment of the invention

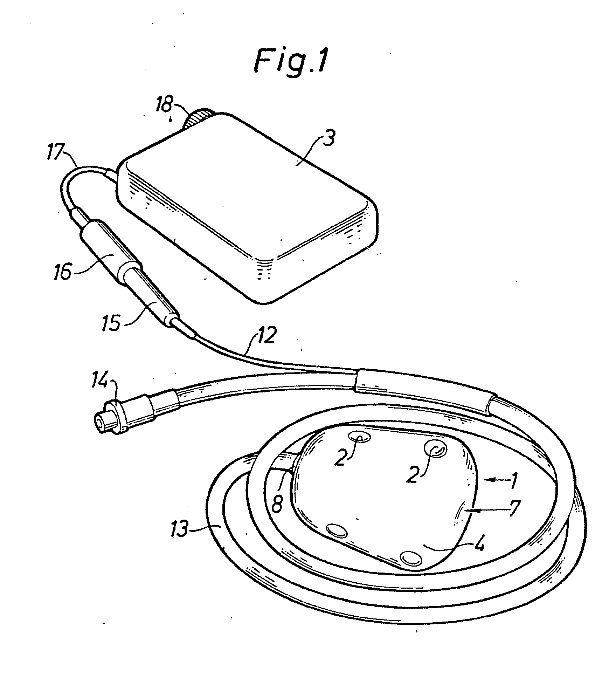

The preferred embodiment of the stimulator in accordance with the invention shown in Fig. 1 thus comprises an element intended for vaginal placing, which as a whole is designated by numeral 1. In the embodiment shown this element carries four electrodes 2 which are adapted so as to control through electrical pulses from the pulse generator 3, shown schematically, the urethral, urocystic and/or rectal functions.

An essential feature of the invention is in the first place the shape of the said element 1 which consists substantially of a bladder 4 made of rubber, plastics or some other expandable material. At the same time the element must also be completely water-tight.

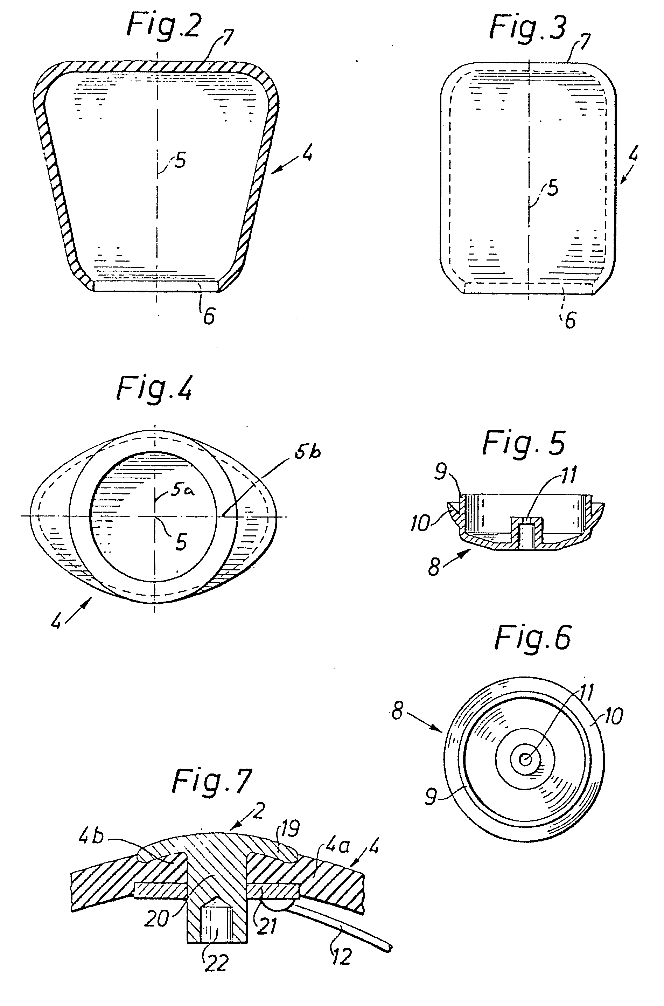

The shape of the bladder 4 can best be seen from Fig. 2—4. It can be said to have a longitudinal axis 5, even though the length does not have to exceed its greatest thickness. At right angles to the axis 5, the cross-section is substantially oval-shaped with a shorter axis 5a and a longer axis 5b, except closest to the open mouth 6, where the cross-section is substantially circular. The inside end in its position of application is shaped with a substantially plane end surface 7. The opposite end of the bladder 4 is closed by an end piece 8, which can be seen in more detail in Fig. 5 and 6. This end piece 8 is provided with a circular-cylindrical flange 9 which is intended to be attached inside the bladder 4 and a substantially conical flange 10 which is intended to surround the mouth or opening 6 of the bladder 4. The end piece 8 is provided moreover with a small opening 11 for the introduction of leads 12 connected to the electrodes 2 and for the connection of a tube 13 for the inflation and evacuation respectively of the element 1.

As can be seen from Fig. 1, the tube 13 ends in a coupling component 14 which is adapted so that it can be connected to a simple hand pump for either inflation or evacuation of the element 1. In the same manner the leads 12, which in Fig. 1 are shown as a conventional two-wire cable, terminate in an electrical coupling component 15 which is joined to a second corresponding component 16 which is connected to the pulse generator 3 via a cable 17. Finally, numeral 18 in Fig. 1 designates a control knob which may be adapted for the setting of the amplitude of the pulses, the pulse width and/or the frequency.

In Fig. 7 finally is shown an example of the rivetlike electrodes 2 and their attachment. The rivet consists of a head 19 and a shank 20. Between the head 19 and a plate 21 is clamped the wall 4a of the bladder 4. This is realized with strong compression of the wall material 4b between the head 19 and the plate 21. This ensures an effective seal. Numeral 22 designates a recess in the end of the rivet shank 20. This recess is intended for facilitating the assembling of the rivet, as it is important that this assembling should take place in a correct manner, so as to ensure sealing. In Fig. 7 is shown how the plate 21 is fixed firmly to the rivet. In practice, however, this is done appropriately by way of deformation of the rivet shank 20, that is to say, in conventional manner. The lead 12 can be soldered firmly onto the plate 21. Alternatively, it may, of course, also be connected directly to the electrode.

It has been found in practice that the head diameter of the rivetlike electrode should not be less than approx. 6 mm and not be more than approx. 12 mm. With smaller sizes there is a danger of the electrode coming loose, and if they are larger the energy consumption increases. It has also been found that the round shape of the electrodes has special advantages from a medical point of view by better fitting different patients.

**Claims**

1. An electrical stimulator comprising an element (1) intended for vaginal placing, carrying two or more electrodes (2) which are adapted so as to control, through electrical pulses from a pulse generator (3), the urethral and/or urocystic functions, said element (1) having a shape that is not rotational-symmetrical, characterized in that the cross-sections of said element (1) at right angles to an axis (5) which runs parallel with the direction of introduction have an elongated form, the electrodes (2) being arranged on one major surface only of the element on one side of the longitudinal axis (5b) of said cross-sections, the shape of the element (1) enabling it to be placed in the vagina in a rotationally-stable position with the electrodes directed towards the bladder.

2. A stimulator in accordance with claim 1, characterized in that the said element (1) is of substantially oval cross-section at right angles to an axis (5) which runs parallel with the direction of introduction, the area of these cross-sections preferably increasing in the direction of introduction.

3. A stimulator in accordance with claim 1, characterized in that the said element (1) is expandable by inflation.

4. A stimulator in accordance with claim 1, characterized in that the said element (1) is constituted of a bladder (4) made of an expandable materal such as rubber or plastics and which is open at one end (6), and of a rotation-symmetrical end piece (8) which is provided with a smaller opening (11) for the introduction of leads (12) connected to the electrodes (2), and for the connection of a tube (13) for the inflation and evacuation respectively of the element (1).

5. A stimulator in accordance with claim 4, characterized in that said electric leads (12) are attached to plates (21) which in turn are attached to the shanks (20) of rivetlike electrodes (2) whose heads (19) are arranged on the outside of the said element (1), the shanks (20) penetrating the wall (4a) of the element, an effective seal being achieved in that the said wall (4a) is held clamped between the plates (21) and the heads (19).

6. A stimulator in accordance with claim 5, characterized in that the said heads (19) and/or the said plates (21) are of a dished shape facing towards the wall (4a) of the element so as to facilitate sealing.

7. A stimulator in accordance with claim 5, characterized in that the shanks (20) of the rivetlike electrodes have a hollow end (22) to facilitate the assembling of the same.

8. A stimulator in accordance with claim 4, characterized in that the end piece (8) is provided with a circular-cylindrical flange (9) which is attached inside the bladder (4), and a substantially conical flange (10) which is adapted so that it surrounds the mouth (6) of the opening (6) in a sealing manner.

**Patentansprüche**

1. Elektrischer Stimulator mit einem Element (1), welches in einer Scheide angeordnet werden soll und zwei oder mehr Elektroden (2) trägt, die geeignet derart ausgestaltet sind, daß sie über elektrische Impulse aus einem Impulsgenerator (3) die Harnröhren und/oder Harnblasenfuktionen steuern, wobei das Element (1) eine Gestaltung hat, die nicht rotationssymmetrisch ist, dadurch gekennzeichnet, daß die Querschnitte des Elementes (1) unter rechten Winkeln zu einer Achse (5), die parallel zur Einführrichtung verlaufen, eine längliche Form haben, wobei die Elektroden (2) auf nur einer größeren Oberfläche des Elementes auf einer Seite der Längsachse (5b) der Querschnitte angeordnet sind und die Gestalt des Elementes (1) die Möglichkeit vorsieht, daß dieses in der Scheide in drehstabiler Position angeordnet wird, wobei die Elektroden zu der Blase hin gerichtet sind.

2. Stimulator nach Anspruch 1, dadurch gekennzeichnet, daß das Element (1) im wesentlichen ovalen Querschnitt unter rechten Winkeln zu einer Achse (5) hat, die parallel zur Einführrichtung verlaufen, wobei die Flächen dieser Querschnitte in Einführrichtung vorzugsweise zunehmen.

3. Stimulator nach Anspruch 1, dadurch gekennzeichnet, daß das Element (1) durch Auf-
blasen ausweitbar ist.

4. Stimulator nach Anspruch 1, dadurch gekennzeichnet, daß das Element (1) aus einer
Blase (4) aufgebaut ist, die aus einem ausweitbaren bzw. dehnbaren Material hergestellt ist,
wie z.B. Kautschuk oder Kunststoff, und welches an einem Ende (6) offen ist, sowie aus
einem rotationssymmetrischen Endstück (8),
welches mit einer kleineren Öffnung (11)
versehen ist, für das Einführen von Drähten (12),
die mit den Elektroden (2) verbunden sind, und
für die Verbindung eines Schlauches (13) für
das Aufblasen bzw. Evakuieren des Elementes
(1).

5. Stimulator nach Anspruch 4, dadurch gekennzeichnet, daß die elektrischen Drähte (12)
an Platten (21) angebracht sind, die ihrerseits
an den Schäften (20) nietenförmiger Elektroden (2) angebracht sind, deren Köpfe (19) auf
der Außenseite des Elementes (1) angeordnet
sind, wobei die Schäfte (20) durch die Wand
(4a) des Elementes hindurchdringen und eine
wirksame Dichtung dadurch erreicht wird, daß
die Wand (4a) zwischen den Platten (21) und
den Köpfen (19) eingeklemmt gehalten wird.

6. Stimulator nach Anspruch 5, dadurch gekennzeichnet, daß die Köpfe (19) und/oder die
Platten (21) konkav gewölbte Gestaltung
haben, welche zur Wand (4a) des Elementes hin
gerichtet ist, um das Abdichten zu erleichtern.

7. Stimulator nach Anspruch 5, dadurch gekennzeichnet, daß die Schäfte (20) der nietartigen Elektroden ein hohles Ende (22) haben,
um die Montage desselben zu erleichtern.

8. Stimulator nach Anspruch 4, dadurch gekennzeichnet, daß das Endstück (8) mit einem
kreiszylindrischen Flansch (9) versehen ist, der
auf der Innenseite der Blase (4) angebracht ist,
sowie mit einem in wesentlichen konischen
Flansch (10), der geeignet derart ausgebildet ist,
daß er den Mund (6) der Öffnung (6) in dichtender Weise umgibt.

## Revendications

1. Un Stimulateur électrique comprenant un
élément (1) destiné à être placé dans le vagin,
portant deux électrodes (2) ou davantage qui
sont conçues pour commander, au moyen
d'impulsions électriques venant d'un générateur
d'impulsions (3), les fonctions urétrales et/ou
urocystiques, ledit élément (1) ayant une forme
qui n'est pas symétrique de révolution, caractérisé en ce que les sections transversales dudit
élément (1) perpendiculaires à un axe (5) qui
est parallèle à la direction d'introduction ont une forme allongée, les électrodes (2) étant
disposées sur une surface principale seulement de l'élément sur un côté de l'axe
longitudinal (5b) desdites sections transversales, la forme de l'élément (1) permettant de la
placer dans le vagin dans une position stable en·
rotation avec les électrodes dirigées vers la
vessie.

2. Un stimulateur suivant la revendication 1,
caractérisé en ce que ledit élément (1) a une
section transversale sensiblement ovale perpendiculairement à un axe (5) qui est parallèle à
la direction d'introduction, la surface de ces
sections transversales augmentant de préférence dans la direction d'introduction.

3. Un simulateur suivant la revendication 1,
caractérisé en ce que ledit élément (1) est
dilatable par gonflage.

4. Un stimulateur suivant la revendication 1,
caractérisé en ce que ledit élément (1) est
constitué d'une vessie (4) en matière expansible telle que du caoutchouc ou une matière
plastique qui est ouverte à une extrémité (6), et
d'une pièce d'extrémité (8) symétrique en
rotation qui comporte un orifice (11) plus petit
pour l'introduction de conducteurs (12) reliés
aux électrodes (2), et pour le raccordement d'un
tube (13) pour le gonflage et la purge respectivement de l'élément (1).

5. Un stimulateur suivant la revendication 4,
caractérisé en ce que lesdits conducteurs
électriques (12) sont attachés à des plaques
(21) qui sont elles-mêmes fixées aux queues (20)
des électrodes (2) en forme de rivet dont les
têtes (19) sont disposées du côté extérieur dudit
élément (1), les queues (20) traversant la paroi
(4a) de l'élément, une étanchéité effective étant
obtenue en ce que ladite paroi (4a) est
maintenue bloquée entre les plaques (21) et les
têtes (19).

6. Un stimulateur suivant la revendication 5,
caractérisé en ce que lesdites têtes (19) et/ou
lesdites plaques (21) sont en forme de cuvette
tournée vers la paroi (4a) de l'élément de façon
à faciliter l'étanchéité.

7. Un stimulateur suivant la revendication 5,
caractérisé en ce que les queues (20) des
électrodes en forme de rivet ont une extrémité
creuse (22) pour faciliter l'assemblage des
électrodes.

8. Un stimulateur suivant la revendication 4,
caractérisé en ce que la pièce d'extrémité (8)
comporte une collerette cylindrique circulaire
(9) qui est fixée à l'intérieur de la vessie (4), et
une bride (10) sensiblement conique qui est
prévue pour entourer l'embouchure (6) de
l'orifice (6) de façon étanche.

# Fig.1

*Fig.2*

*Fig.3*

*Fig.4*

*Fig.5*

*Fig.6*

*Fig.7*

2